Europäisches Patentamt

European Patent Office

Office européen des brevets

(11) Publication number: **0 370 700**
**A1**

## (12) EUROPEAN PATENT APPLICATION

(21) Application number: 89311875.2

(22) Date of filing: 17.11.89

(51) Int. Cl.5: **G01N 33/579**

(30) Priority: 25.11.88 GB 8827557

(43) Date of publication of application:
**30.05.90 Bulletin 90/22**

(84) Designated Contracting States:
**CH DE ES FR GB IT LI**

(71) Applicant: SENSITITRE LIMITED
The Manor Manor Royal
Crawley W. Sussex RH10 2PY(GB)

(72) Inventor: Hill, Simon Julian Roland
45 Sackville Gardens
East Grinstead W. Sussex(GB)

(74) Representative: Jones, Helen Marjorie
Meredith et al
Gill Jennings & Every 53-64 Chancery Lane
London WC2A 1HN(GB)

(54) Process for handling horseshoe crab amoebocyte lysate.

(57) Horseshoe crab amoebocyte lysate is stabilised during its handling in aqueous dispersion by buffering it at a pH outside the range 6-8. The lysate is subsequently used in a process for detecting endotoxin in an assay sample in solution at a pH in the range 6-8.5.

EP 0 370 700 A1

## PROCESS FOR HANDLING HORSESHOE CRAB AMOEBOCYTE LYSATE

The present invention relates to the handling of the amoebocyte lysate of horseshoe crab, or an extract thereof which contains the pro-clotting enzyme, in a manner which stabilises the lysate so that it is not prematurely activated.

The amoebocyte lysate of horseshoe crabs is widely used as a very sensitive assay to test for the presence and amount of bacterial endotoxin in samples. The gelation reaction involves a cascade of enzymic reactions which is triggered by endotoxins. It can be observed by assessing turbidity changes, (US 4038029), by analysing the creation of a plug by the clot in a capillary tube (US 4370413) or by testing the depth of penetration of objects dropped onto the surface of an assay mixture. More recently synthetic substrates have been developed which are hydrolysed by one or more of the active enzyme products formed during the clotting process to produce for instance coloured compounds or fluorescent compounds. For instance US 4188264 discloses a range of fluorogenic and chromogenic substrates for the horseshoe crab amoebocyte lysate assay. In EP 80649 biological fluids, including urine, are analysed using synthetic peptide substrates for the amoebocyte lysate. The products of which can be detected in the mixture. The product can be observed directly, by observing its colour, or can be converted to another compound in the mixture, e.g. by coupling with a coupling reagent to form an azo dye which has a more easily observable colour. Nitroanilide derivatives can be observed directly by their colour and are preferred. Fluorogenic substrates are also mentioned. In EP 224830 chromogenic or fluorogenic substrates with amoebocyte lysate of horseshoe crab are used in a urine screen.

In early disclosures of the production of lysate from crabs, the lysate was often stored in the form of an aqueous solution, usually the solution produced in the lysing step. For instance in US 3915805 the lysate in artificial sea water or distilled water is stored in the liquid form at 4°C at which it is said to be stable for many months. In US 4038147 the lysate formed by lysing the cells in pyrogen free water is stored at around 7°C at which is said to be stable for about a year.

Subsequently, as disclosed in GB 1522127. it has been found that improved storage stability, i.e. improved retention of clotting ability could be achieved by lyophilising or freeze-drying the lysate. The lyophilisate powder is then stored. Generally it is the solution formed in the lysate reaction which is lyophilised. For instance in US 4107077 and in GB 1522127 the amoebocytes are lysed using pyrogen free water and the lysate is immediately lyophilised.

The lysate solution may sometimes be buffered before being lyophilised. In all disclosures the buffer is chosen so that when reconstituted the lysate will have a pH within the active range of 6 to about 9. Thus in EP 747161 the lysate is buffered in the range 6.5 to 7.5 before lyophilisation. In US 4038029 the lysate is buffered at a pH in the range 6.5 to 6.8 before lyophilisation. In US 4322217 it is stated that the lysate should be buffered within the active pH range and preferably 6.5 to 7.5, before lyophilisation. It is mentioned in the last reference that the lysate could be buffered to within the range 5.5 to 8.5, although it is quite clear in the context that the pH range is intended to be that within which the lysate is active.

In EP-A-0224830 solid state devices of two types are disclosed. In the first the lysate appears to be buffered in the range pH 6.3 to 7.5 before being dried and in the second embodiment the lysate is buffered at a pH in the range 7.5 to 8.5.

In US 4301245 the stability of lysate is improved, by preventing premature gelation by the addition of heparin to the lysate prior to lyophilisation. it is thought that the clotting cascade requires the presence of calcium and/or other disalent cations and the incorporation of heparin may have some effect on any disalent cations in the in the lysate, interfering with the cascade.

The problem with all the methods of stabilising the lysate in the prior art is that the improvement in storage life and stability during other handling operations is still unsatisfactory.

Young et al in J. Clin. Invest (1972) 51 1790 test the pH sensitivity of the gelation reaction and find that at pH 5.7 or 8.6 there is little gelation whereas between these values there is gelation. In EP-A-80649 it is stated that the pH in the reaction mixture should preferably be between 6 and 7 for observation by gelation and pH 7 to 9 for observation using a synthetic hydrolysable substrate.

There have been various disclosures of methods of treating the lysate to remove inhibitors. Many of these rely on solvent treatment, which is thought to denature an inhibitor. Such a process is described in US 4107077, described above. In US 4376819 a hydrolase contaminant is removed from the lysate by raising the pH to a value of above 8.5 and then lowering the pH again to within the active range. The pH is said to be preferably raised to within the range 10.5 to 11.0. In one embodiment of the process the pH is raised by the addition ammonium carbonate and the solution is then lyophilised without prior neutralisation, and during the lyophilisation ammonia volatilises so that there is an in situ lowering of the pH. There is no working example of using such a process however and the sole example uses sodium hydroxide to raise the pH and hydrochloric acid to lower it again before lyophilisation.

It is known that acidification of lysate inactivates the gelation reaction. In Thrombosis Research (1973) 2 55 to 70 Solum describes the separation of lysate into various protein fractions. One fraction of lysate protein is precipitated by lowering the pH to around 1.6. The supernatent, containing the molecular weight proteins, contain the coagulogen. This cannot be gelled even at the pHs at which the lysate is normally active, unless a portion of untreated lysate or trypsin is added. This suggests that the acidification technique either removes an enzyme of the cascade completely or irreversably inactivates it.

We have now found that by a careful adjustment of the pH of aqueous lysate there can be a great improvement in the stability.

According to the present invention a new process for handling horseshoe crab amoebocyte lysate or a pro-clotting enzyme containing extract thereof in aqueous dispersion before use of the lysate of extract in a process for assaying a sample for endotoxin comprises maintaining the pH of the dispersion at a value before 6.0 or above 8.0 during said handling.

Preferably the pH is maintained in the quoted ranges throughout the handling operation.

The lysate is subsequently used in an assay for detecting the presence of endotoxin in liquid samples. The lysate will in such assays be used at a pH within its active range, that is in the range 6.0 to 8.5, and so the pH of the lysate is adjusted to within that range.

Generally the aqueous dispersion of lysate is buffered at the required pH level. In the subsequent assay the pH is generally adjusted to within the active range by the incorporation of another buffer. Thus where the lysate dispersion is handled in the process of the invention at a pH of less than 6, it may be subsequently be contacted with another buffer, having a pH of above 6, for instance above 7 or 8, to give an assay solution having a pH between that of the two buffers.

It is particularly preferred for the pH of the lysate dispersion during the handling process to be on the acidic side of the active pH. It is thus preferably less than 6.0. The pH should be sufficiently high that the proenzyme is not adversely affected, for instance is not denaturised or precipitated. The pH should thus preferable be above 3 or 4, and often does not need to be lower than 4.5 or 5.0. Satisfactory results are often achieved when the pH is above 5.5, for instance in the range 5.5 to 5.9, most preferably 5.7 to 5.9.

When the pH is alkaline it is usually more than 8.5. The lysate is then reconstituted in an acidic buffer to give a pH within which the lysate is active.

Preferably the handling is carried out at low temperatures since this further reduces the likelihood of the lysate being prematurely activated. The temperature is thus preferably less than 10°C more preferably less than 5°C. The dispersion can also be frozen and thawed if necessary.

The lysate may be stored in the dispersion and thus the handling step may include a storage step. It is often more convenient for the lysate to be stored in dried form and thus the dispersion may be dried by conventional techniques. It may for instance be air dried, vacuum dried or, most preferably, lyophilised. When the lysate is dried the dispersion conveniently contains other physical or chemical stabilisers, for instance water-soluble film forming stabilisers such as natural or synthetic polymers, for instance gelatin or polyvinyl alcohol, or other stabilisers of the type described in GB 1522127 or may contain other additives such as those described in US 4273557 and US 4322217.

We have found that the process of the invention is of particular value where the lysate dispersion is reconstituted from previously dried, generally freeze-dried, form for subsequent use. The pH controlled LAL is far more stable than non-pH controlled LAL having adequate stability that it can be retained for a whole working day, i.e. about 7 hours, usually at reduced temperatures, such as around 4°C. In contrast non pH controlled LAL has very variable stability and is extremely sensitive to even very low levels of contamination which alter its performance.

The invention is further illustrated in the accompanying example.

EXAMPLE 1

COMPARATIVE

Freeze-dried LAL in an amount which is suitable for preparing 5ml of a 0.125 Eu/ml sensitivity solution (produced by Associates of Cape Cod) is dissolved in 160ml of 0.05% solution of polyvinylalcohol (cold water soluble produced by Sigma). The pH of the solution is 6.9. 50µl of the solution is dosed into the wells and the solutions vacuum dried.

INVENTION

A pH5.8 buffer was prepared by mixing 2 parts (by volume) of a 0.1M solution of citric acid in pyrogen free water with 3 parts of a 0.2M solution of disodium hydrogen orthophosphate. Freeze dried LAL in an amount which is suitable for preparing 5 ml of a 0.125 Eu/ml sensitivity solution (produce by Whittaker) was re-dissolved in 10 ml of a 0.1% polyvinylalcohol solution. To this was added 10 ml of the pH5.8 buffer. 50μl portions of the solution were dosed into wells of a microtitre plate and vacuum dried.

## ASSAY

The plates were subsequently used in an assay for detecting the presence of endotoxin in urine as part of a urine screen. The dried LAL was reconstituted by the addition of 50μl of a substrate solution. The substrate solution was made up by mixing 250 ml pyrogen free water, 1.25g sodium hydroxide, 9.38g TES biological buffer (Sigma) 2.5g polyvinyl alcohol (as above) and 1.25g magnesium chloride. The mixture is heated to dissolve the ingredients, adjusted to pH 8.00 ± 0.05 using pyrogen free 0.1 MHC1 or 0.1 MNaOH. The solution is then filter sterilised. Separately a solution of Boc-Val-Gly-Arg-AMC is made up to 0.1% w/v in 2-methoxyethanol. 300μl of that solution is mixed with 9.7ml of the buffer. The pH of the reconstituted solution is about 7.3.

1μl urine is dosed into each well to start the assay. The plate is covered with an adhesive sheet seal and incubated at 37°C for 30 minutes, after which the presence of 7-amino-4methyl-coumarin is detected in each of the wells fluorimetrically by an automatic reader. The readings are compared with the neat urine reading in blank wells. The tests register positive when the urine contains more than $10^4$ cfu/ml bacteria.

## STABILITY

The plates which are produced using the process of the invention are found to give highly reproducible results when reconstituted whereas the plates of the comparative example do no longer produce reliable results. This is mainly due to the loss of activity of the lysate during the production procedure prior to the lyophilisation in the comparative example because of difficulties in completely freezing the apparatus used from endotoxin.

## EXAMPLE 2

In another embodiment of the invention, the following solutions are made up. 50μl portions of a substrate solution as in the previous example, but containing also 0.05% poly-vinyl alcohol and buffered at pH8.00 ± 0.05 were dosed into wells of a plate and dried. A buffered pH5.8 lysate solution, was made up immediately before use of the kit, by the same method as example 3, but omitting the polyvinyl alcohol.

The wells are reconstituted by adding 50μl portions of the lysate solution to the substrate containing wells to give a pH of about 7.3, and then 1μl urine is added in the same way as in the preceding example to commence the reaction.

## Claims

1. A process for handling horseshoe crab amoebocyte lysate or a proclotting enzyme containing extract thereof in aqueous dispersion before its use in a process for assaying a sample for endotoxin, in which the pH of the aqueous solution is maintained at a value below 6.0 or at a value above 8.0 during said handling.

2. A process according to claim 1 in which the pH is maintained in the stated ranges substantially throughout said handling step.

3. A process according to claim 1 or claim 2 in which the dispersion is buffered at the specified pH.

4. A process according to any preceding claim in which the lysate or extract is subsequently used in an assay in aqueous solution at a pH in the range 6.0 to 8.5, the lysate being subjected to pH in this range from immediately prior to the start of the assay.

5. A process according to claim 4 in which the pH in the assay solution is created by mixing the lysate containing a buffer to maintain its value below 6 or above 8.0 is contacted with a buffer having a pH above 6 or below 8.0, respectively.

6. A process according to any preceding claim in which the aqueous dispersion is maintained at a pH in the range 4.0 to 6.0, preferably 4.5 to 5.9, more preferably 5.5 to 5.9, most preferably 5.7 to 5.9, during said handling.

7. A process according to any of claims 1 to 5 in which the aqueous dispersion is maintained at a pH of above 8.5 during said handling.

8. A process according to any preceding claim in which after the handling the dispersion is subsequently dried, preferably by vacuum, air or, most preferably freeze drying, optionally in the presence of a stabiliser.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | GB-A-2 080 524 (MALLINCKRODT INC.) * Page 1, lines 46-53 * & US-A-4 322 217 (Cat. D) --- | 1-5,7 | G 01 N 33/579 |
| A | H.U. BERGMEYER: "Methods of Enzymatic Analysis", 3rd edition, vol. XI, 1986, pages 102-11, Verlag Chemie, Weinheim, DE * Pages 102,108 * --- | 4,5 | |
| D,A | US-A-4 376 819 (R. CLARK BROWN et al.) * Abstract; column 4, line 22 - column 5, line 69 * --- | 1-5,7 | |
| P,X | EP-A-0 320 154 (RADIOMETER CORPORATE DEVELOPMENT LTD) * Page 6, lines 29-35; page 8, lines 25-46 * ----- | 1-8 | |
| | | | TECHNICAL FIELDS SEARCHED (Int. Cl.5) |
| | | | G 01 N |

The present search report has been drawn up for all claims

| Place of search | Date of completion of the search | Examiner |
|---|---|---|
| THE HAGUE | 06-03-1990 | GRIFFITH G. |

EPO FORM 1503 03.82 (P0401)